# EUROPÄISCHE PATENTSCHRIFT

(11) **EP 0 617 007 B1**
(45) Veröffentlichungstag und Bekanntmachung des Hinweises auf die Patenterteilung: **08.01.1997**
(21) Anmeldenummer: 94103291.4
(22) Anmeldetag: 04.03.1994
(51) Int. Cl.: C07C 221/00, C07C 225/14, C07D 295/10

(54) **Verfahren zur Herstellung von 1-Aminovinylmethylketonen**
Method for the preparation of 1-aminovinyl methyl ketones
Procédé pour la préparation des 1-aminovinyl méthyl cétones

(30) Priorität: 13.03.1993 DE 4308080
(43) Veröffentlichungstag der Anmeldung: 28.09.1994
(73) Patentinhaber: BASF Aktiengesellschaft, 67063 Ludwigshafen (DE)
(72) Erfinder: Rittinger, Stefan, Dr., D-67059 Ludwigshafen (DE); Rieber, Norbert, Dr., D-68259 Mannheim (DE)

(56) Entgegenhaltungen:
- DE-C- 897 565
- CHEMICAL ABSTRACTS, vol. 79, no. 19, 12. November 1973, Columbus, Ohio, US; abstract no. 115031j, A. N. VOLKOV ET. AL. 'Diacetylene derivatives. XXVI. Reaction of diacetylene with aqueous solutions of amines.' Seite 314 ;Spalte 1 ;
- ZEITSCHRIFT FüR CHEMIE Bd. 9, Nr. 3 , 1969 , LEIPZIG, DE Seiten 108 - 9 W. SCHROTH ET. AL. 'Reaktion von Diacetylen mit Amine'

## Beschreibung

Die vorliegende Erfindung betrifft ein Verfahren zur Herstellung von 1-Aminovinylmethylketonen aus Diacetylen, sekundärem Amin und Wasser.

Aus Z. Chem. 9, 109 (1969) ist ein Verfahren zur Herstellung von Aminovinylmethylketonen aus Diacetylen, sekundären Aminen und Wasser im Labormaßstab bekannt.

Aus DE-A-12 22 910 ist jedoch bekannt, daß die Handhabung von Diacetylen im technischen Maßstab äußerst problematisch ist und daher nach EP-A-274 600 nahezu ausschließlich zur Herstellung von Laborpräparaten in entsprechend kleinen Mengen genutzt wird.

Der vorliegenden Erfindung liegt daher die Aufgabe zugrunde, den zuvor genannten Nachteilen abzuhelfen.

Demgemäß wurde ein neues und verbessertes Verfahren zur Herstellung von 1-Aminovinylmethylketonen der allgemeinen Formel I 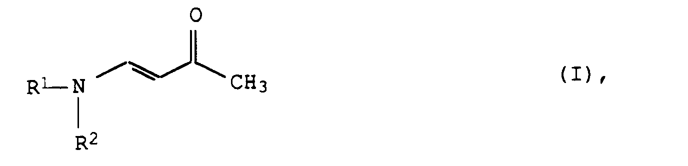
in der R¹ und R² unabhängig voneinander C₁- bis C₂₀-Alkyl, C₃- bis C₈-Cycloalkyl, C₁- bis C₂₀-Hydroxyalkyl, Aryl, C₇- bis C₁₂-Phenalkyl, C₇- bis C₁₂-Alkylphenyl oder gemeinsam eine gegebenenfalls ein- bis vierfach durch C₁ - bis C₄-Alkyl substituierte gegebenenfalls durch Sauerstoff, Stickstoff oder Schwefel unterbrochene C₂- bis C₇-Alkylenkette

bedeuten, aus Diacetylen und sekundären Aminen, gefunden, welches dadurch gekennzeichnet ist, daß man
a) aus dem bei der Acetylenerzeugung resultierenden Spaltgas einen Teilstrom, der Diacetylen der Formel II

   H-C≡C-C≡C-H (II),

   enthält, durch Absorption abtrennt und
b) diesen Teilstrom mit sekundären Aminen der allgemeinen Formel III 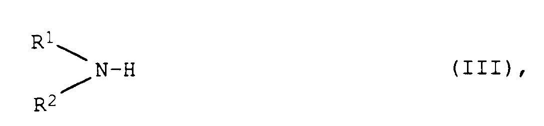 in der R¹ und R² die obengenannten Bedeutungen haben, und Wasser gegebenenfalls in einem Verdünnungsmittel bei Temperaturen von 0 bis 150°C und Drücken von 0,01 bis 5 bar umsetzt.

Das erfindungsgemäße Verfahren läßt sich wie folgt durchführen:

Das - bei der Zerlegung von Spaltgasen aus der Spaltung von Kohlenwasserstoffen unter den Bedingungen der Acetylensynthese als verdünnter Teilstrom - anfallende Gemisch höherer Acetylene kann ohne weitere physikalische oder chemische Behandlung unmittelbar mit wäßrigen Lösungen von sekundären Aminen bei Temperaturen von 0 bis 150°C, bevorzugt 30 bis 130°C, besonders bevorzugt 40 bis 120°C und Drücken von 0,01 bis 5 bar, bevorzugt 0,1 bis 2,5 bar, besonders bevorzugt 0,5 bis 1,5 bar, in der Regel bei Normaldruck (Atmosphärendruck), umgesetzt werden.

Die technischen Varianten zur Erzeugung und Aufbereitung der erfindungsgemäß verwendeten Spaltgase ("Crack-Gase") sind z.B. aus Ullmans Encyclopedia of Industrial Chemistry, V. Edition, A1, 1985, Seite 97 bis 145, insbesondere Seite 111, Fig. 13 bekannt, wobei Kohlenwasserstoffe (z. B. Erdgas oder auch höhersiedende Fraktionen) bei den für die Acetylenentstehung notwendigen, hohen Temperaturen gespalten und die resultierenden Produkte unmittelbar nach der Reaktionszone (Spaltzone) durch Eindüsen einer Flüssigkeit (z. B. Wasser oder Öl) rasch abgekühlt ("Quench") werden. Die Zusammensetzung der Spaltgase ist abhängig von den zur Spaltung eingesetzten Ausgangsstoffen, sowie den Spaltungsbedingungen.

Die erfindungsgemäß verwendeten Teilströme fallen bei der Zerlegung des Spaltgases durch eine Serie von physikalischen Trennvorgängen, bevorzugt durch Absorptions-/Desorptionsprozesse in einer Serie von Wasch- und Stripkreisläufen, in mehreren Teilströmen an. Die verwendbaren, höhere Acetylene enthaltenden Teilströme haben gegenüber dem Spaltgas deutlich höhere Konzentrationen an Diacetylen.

Eine weitere Aufbereitung des Einsatzstromes gemäß DE-A-21 57 537 ist nicht notwendig. Sowohl flüssige Einsatzströme als auch gasförmige, durch Strippen (Desorption) der Waschlösungen erhältliche Teilströme zeichnen sich gegenüber dem Spaltgas durch ein für die gewünschte Umsetzung zu Aminovinylmethylketon günstigeres Verteilungsprofil der höheren Acetylene aus. Es sind nur noch unwesentliche Mengen anderer, höherer Acetylene (Pentadiin, Hexadiin, etc.) enthalten, da diese vorher abgeschieden werden. Dies führt zu einer hohen Reinheit des erhaltenen Produktes.

Die Abtrennung der höheren Acetylene aus dem Spaltgas erfolgt in bevorzugter Weise so, daß die höheren Acetylene in einer Vorwäsche des das Gesamtacetylen enthaltenden Spaltgases mit einer Flüssigkeit absorbiert werden. Diese mit Diacetylen angereicherte Waschlösung kann dann noch in einem Vakuumstripper entgast werden, wobei die Gasfraktion nochmals vor oder unmittelbar nach einer eventuellen Kompression durch Zumischen eines Inertgasanteils stabilisiert wird.

Diacetylenhaltige Kohlenwasserstoffkondensate (sog. BTX-Fraktion) aus diesem Gasstrom eignen sich gleichermaßen vorteilhaft für die beschriebene Umsetzung von Diacetylen zu 1-Aminovinylmethylketon. Dies bewirkt für diese Vorgehensweise den zusätzlichen Vorteil, daß nach Abreaktion des Diacetylens destillativ die von Diacetylen befreite BTX-Fraktion erhalten werden kann, welche beispielsweise wieder als Einsatzstoff zur Crackung verwendet werden kann.

Als Absorptionsmittel in der Spaltgasaufbereitung eignen sich Wasser, hochsiedende Kohlenwasserstoffe wie Aromatengemische bevorzugt Rückstandsöl, N-Alkyllactame mit C₁- bis C₃-Alkyl, bevorzugt N-Methylpyrrolidon, C₁- bis C₅-Alkohole, bevorzugt Methanol, Säureamide, bevorzugt Dimethylformamid, alkylierte cyclische Harnstoffe, bevorzugt Dimethylpropylenharnstoff, primäre, sekundäre und tertiäre C₁- bis C₆-Amine und Ammoniak.

Zur Verdünnung der aus dem Absorbens entfernten diacetylenhaltigen Gase eignen sich Inertgase wie Kohlenwasserstoffe, Kohlenmonoxid, Synthesegas, Armgas, Stickstoff oder Gemische dieser Gase, bevorzugt Erdgas und/oder Acetylen.

Das inertisierte Gasgemisch enthält in der Regel bevorzugt 55 bis 85 Vol-% Inertgas, 1 bis 30 Vol-% Diacetylen und 10 bis 20 Vol-% andere Bestandteile wie Acetylen, Vinylacetylen und Benzol. Der Anteil des Diacetylens im inertisierten Gasgemisch beträgt in der Regel 1 bis 30 Vol-%, bevorzugt 5 bis 20 Vol-%. Die Obergrenze wird durch die Grenze des explosiven Selbstzerfalls im Inertgas bestimmt.

Typische gasförmige, höhere Acetylene enthaltende Teilströme haben folgende Zusammensetzung:
- 58 Vol-% Methan, 18 Vol-% Diacetylen, 5 Vol-% Stickstoff, 4 Vol-% Acetylen, 4,5 Vol-% Vinylacetylen, 4 Vol-% Benzol, 2 Vol-% Ethan, 2 Vol-% Cyclopentadien, 2,5 Vol-% Restbestandteile.

Typische flüssige, höhere Acetylene enthaltende Teilströme haben folgende Zusammensetzung:
- 32 Vol-% Benzol, 28 Vol-% Toluol, 17 Vol-% Xylol (BTX), 8 Vol-% Styrol, 6 Vol-% Diacetylen,
- Methanol (89 Vol-%), Acetylen (0,6 Vol-%), Diacetylen (2,2 Vol-%), Vinylacetylen (0,4 Vol-%), Cyclopentadien (1 Vol-%), Benzol (1,8 Vol-%) und Toluol (1,3 Vol-%).

Die Umsetzung des Teilstroms mit wäßrigen sekundären Aminen kann sowohl diskontinuierlich als auch kontinuierlich gestaltet werden. Bei Nutzung eines gasförmigen Teilstroms sind Verfahren, die eine gute Gasverteilung in Flüssigkeiten erzielen, z.B. die Verwendung von Apparateteilen wie Begasungsring, Lochboden, verdichtende Begasungseinrichtungen, Sprühreaktor oder Absorberturm vorteilhaft.

Nicht vollständig umgesetztes Amin kann destillativ vom Produkt oder dem bei unmittelbarer Weiterverarbeitung erhältlichen Folgeprodukt abgetrennt und in die Synthesestufe a) zurückgeführt werden.

Das Gasgemisch aus der Spaltgasaufbereitung hat gewöhnlich gegenüber der Umgebung leicht erhöhte Betriebstemperatur. Die Vorabscheidung von leicht kondensierbaren Gasbestandteilen in Abscheidebehältern in der Reaktorzuleitung bei Umgebungstemperatur verbessert die Reinheit des Rohproduktes.

Die Substituenten R¹ und R² in den Verbindungen I und III haben folgende Bedeutungen:
- unabhängig voneiander
- C₁- bis C₂₀-Alkyl, bevorzugt C₁- bis C₈-Alkyl, besonders bevorzugt C₁- bis C₄-Alkyl wie Methyl, Ethyl, n-Propyl, iso-Propyl, n-Butyl, iso-Butyl, sec.-Butyl und tert.-Butyl,
- C₃- bis C₈-Cycloalkyl, bevorzugt C₅- bis C₈-Cycloalkyl wie Cyclopentyl, Cyclohexyl, Cycloheptyl und Cyclooctyl, besonders bevorzugt Cyclopentyl und Cyclohexyl,
- C₁- bis C₂₀-Hydroxyalkyl, bevorzugt C₂- bis C₈-Hydroxyalkyl, besonders bevorzugt C₂- bis C₄-Hydroxyalkyl wie 2-Hydroxyethyl und 3-Hydroxypropyl,
- Aryl wie Phenyl, 1-Naphthyl und 2-Naphthyl, bevorzugt Phenyl, 1-Naphthyl und 2-Naphthyl, besonders bevorzugt Phenyl,
- C₇- bis C₁₂-Alkylphenyl wie 2-Methylphenyl, 3-Methylphenyl, 4-Methylphenyl, 2,4-Dimethylphenyl, 2,5-Dimethylphenyl, 2,6-Dimethylphenyl, 3,5-Dimethylphenyl und 2,3,4-Trimethylphenyl,
- C₇- bis C₁₂-Phenylalkyl wie Benzyl, 1-Phenethyl, 2-Phenethyl, 1-Phenyl-propyl, 2-Phenyl-propyl, 1-Phenyl-butyl, 2-Phenyl-butyl, 3-Phenyl-butyl, bevorzugt Benzyl, 1-Phenethyl und 2-Phenethyl,
- gemeinsam
- eine gegebenenfalls durch ein bis vier C₁- bis C₄-Alkylreste substituierte gegebenenfalls durch Sauerstoff, Stickstoff oder Schwefel unterbrochene C₂- bis C₇-Alkylenkette wie -(CH₂)₂-, -(CH₂)₃-, -(CH₂)₄-, -(CH₂)₅-, -(CH₂)₆-, -(CH₂)₇-, -(CHCH₃)-, -(CH₂)₂-O-(CH₂)₂-, -(CH₂)₂-N-(CH₂)₂-, -(CH₂)₂-S-(CH₂)₂-.

1-Aminovinylketone sind vielseitig verwendbare Zwischenprodukte beispielsweise zur Herstellung von Heterocyclen als Vorprodukte für Pflanzenschutzmittel (DE-A-40 31 723; EP-A-320 750; EP-A-286 968; US-A-3,141,880) oder von Aminoalkoholen (US-A-3,325,491).

### Beispiele

In den folgenden Beispielen ist das inertisierte, diacetylenhaltige Gasgemisch mit HA-Gas und Diacetylen mit DA bezeichnet. Das diacetylenhaltige Absorbat aus der Spaltgasvorwäsche wird mit Vorwaschlösung (und Angabe des Lösungsmittels) benannt. Die diacetylenhaltige Lösung aus dem Sumpf der HA-Gaskühler wird mit BTX-Lösung abgekürzt. Das Diacetylen im HA-Gas bzw. im Abgas nach der Reaktion wurde gaschromatographisch auf einer gepackten Säule (20 % Reoplex 400 auf Chromosorb PAW) mit Trägergas N₂ (35 ml/min.) und FID-Detektion bestimmt. Die Konzentrationen sind in Vol-% angegeben. Die Diacetylenbestimmung in flüssiger Phase sowie die Ermittlung der 1-Aminovinylmethyl- und Reaktandenkonzentrationen erfolgte gaschromatographisch auf einer Kapillarsäule HP1 mit einem WLD-Detektor.

### Beispiel 1

In einer 5 l-Blasensäule mit einer Gaseinleitungsfritte aus Glas (40 bis 90 m Porendurchmesser, D 2) wurden eine Mischung aus 1333 g Morpholin und 666 g H₂O vorgelegt und auf 80°C erhitzt. 140 bis 150 l/h eines Teilstroms des HA-Abgases der Acetylenproduktion wurden während 36 Betriebsstunden durch die Reaktionslösung hindurchgeleitet. Die Diacetylenkonzentration des HA-Gases am Reaktoreingang schwankte von 3 bis 10 %. Bei einer durchschnittlichen Abreicherung des Diacetylens von über 90 % wurden 1-Morpholinovinylmethylketon in einer Ausbeute von 87,5 % erhalten (Gehalt im Reaktoraustrag: 67,1 Gew.%, 2920 g). Der Restmorpholingehalt betrug 1,3 %. Die destillative Fraktionierung des Rohaustrages lieferte 1560 g Reinprodukt als braunschwarze, viskose Flüssigkeit (Sdp.: 126 bis 128°C/0,08 mbar).

### Beispiel 2

Wie in Beispiel 1 wurden in eine mit 1000 g 81%iger wäßriger Dipropylaminlösung gefüllten 2 l-Blasensäule ein HA-Gasstrom von 100 l/h eingeleitet. Die Reaktionstemperatur betrug 60°C und die Reaktion wurde bis zur vollständigen Umsetzung des Amins gefahren. Die Diacetylenkonzentration im HA-Gas schwankte zwischen 12 und 18 % und wurde durchschnittlich zu über 80 % abgereichert. Bei der destillativen Aufarbeitung wurden in 67%iger Ausbeute Cis-1-dipropylaminovinylmethylketon (Sdp.: 132 bis 134°C/0,1 mbar) und in ca. 9%iger Ausbeute das höhersiedende Isomere 3-Dipropylaminobut-2-en-1-al (Sdp.: 145°C/0,1 mbar) erhalten.

### Beispiel 3

In einer 250 ml fassenden Rührapparatur mit Rückflußkühlung wurden 65 g 40%ige Dimethylaminlösung in Wasser und 50 ml Tetrahydrofuran vorgelegt und auf 0°C gekühlt. 60 l HA-Gas mit einem Diacetylengehalt von ca. 10 % wurden binnen 4 h eingeleitet. Anschließend wurde noch ca. 3 h bei 60°C gerührt. Die schwachgelbe Lösung verfärbte sich rotbraun. Der Gehalt an Dimethylaminovinylmethylketon betrug 17 % entsprechend einer Ausbeute von ca. 65 % bezogen auf eingeleitetes Diacetylen und 76 % bezogen auf umgesetztes Dimethylamin.

## Patentansprüche

1. Verfahren zur Herstellung von 1-Aminovinylmethylketonen der allgemeinen Formel I 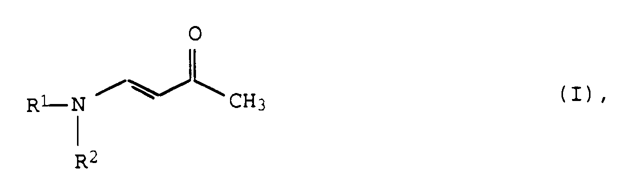 in der R¹ und R² unabhängig voneinander C₁- bis C₂₀-Alkyl, C₃- bis C₈-Cycloalkyl, C₁- bis C₂₀-Hydroxyalkyl, Aryl, C₇- bis C₁₂-Phenalkyl, C₇- bis C₁₂-Alkylphenyl oder gemeinsam eine gegebenenfalls ein- bis vierfach durch C₁- bis C₄-Alkyl substituierte gegebenenfalls durch Sauerstoff, Stickstoff oder Schwefel unterbrochene C₂- bis C₇-Alkylenkette
bedeuten, aus Diacetylen und sekundären Aminen, dadurch gekennzeichnet, daß man
a) aus dem bei der Acetylenerzeugung resultierenden Spaltgas einen Teilstrom, der Diacetylen der Formel II
H-C≡C-C≡C-H (II),
enthält, durch Absorption abtrennt und
b) diesen Teilstrom mit sekundären Aminen der allgemeinen Formel III 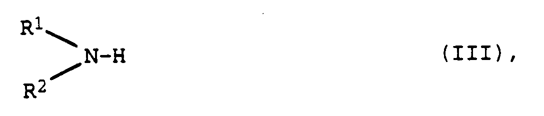 in der R¹ und R² die obengenannten Bedeutungen haben, und Wasser gegebenenfalls in einem Verdünnungsmittel bei Temperaturen von 0 bis 150°C und Drücken von 0,01 bis 5 bar umsetzt.

2. Verfahren zur Herstellung von 1-Aminovinylmethylketonen I nach Anspruch 1, dadurch gekennzeichnet, daß man die Absorption reversibel in einer polaren Flüssigkeit durchführt und gegebenenfalls mit einem Inertgas verdünnt.

3. Verfahren zur Herstellung von 1-Aminovinylmethylketonen I nach Anspruch 1, dadurch gekennzeichnet, daß der Anteil an Diacetylen in verdünnten Teilstrom bei der Umsetzung mit den sekundären Aminen III 2 bis 30 Vol.-% beträgt.

## Claims

1. A process for preparing 1-aminovinyl methyl ketones of the general formula I 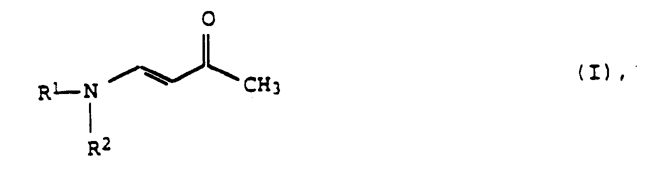 where R¹ and R² independently of one another are C₁- to C₂₀-alkyl, C₃- to C₈-cycloalkyl, C₁- to C₂₀-hydroxyalkyl, aryl, C₇- to C₁₂-phenalkyl, C₇- to C₁₂-alkylphenyl or together are a C₂- to C₇-alkylene chain which may be mono- to tetrasubstituted by C₁- to C₄-alkyl and interrupted by oxygen, nitrogen or sulfur
from diacetylene and secondary amines, which comprises
a) separating off by absorption from the cleavage gas resulting during acetylene generation a part stream which contains diacetylene of the formula II
H-C≡C-C≡C-H (II),
and
b) reacting this part stream with secondary amines of the general formula III 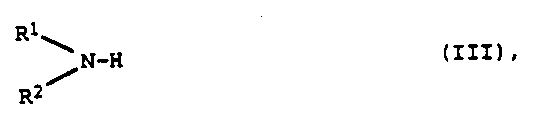 where R¹ and R² have the abovementioned meanings, and water, if appropriate in a diluent at from 0 to 150°C and from 0.01 to 5 bar.

2. A process for preparing 1-aminovinyl methyl ketones I as claimed in claim 1, wherein the absorption is carried out reversibly in a polar liquid and dilution is carried out, if appropriate, with an inert gas.

3. A process for preparing 1-aminovinyl methyl ketones I as claimed in claim 1, wherein the proportion of diacetylene in the dilute part stream during the reaction with the secondary amines III is from 2 to 30% by volume.

## Revendications

1. Procédé de préparation de 1-aminovinylméthylcétones de formule générale I 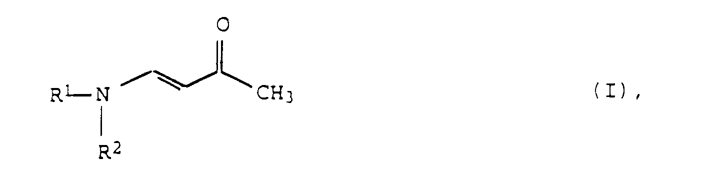 dans laquelle R¹ et R² représentent chacun, indépendamment l'un de l'autre, un reste alkyle en C₁-C₂₀, cycloalkyle en C₃-C₈, hydroxyalkyle en C₁-C₂₀, aryle, phénalkyle en C₇-C₁₂, alkylphényle en C₇-C₁₂, ou représentent ensemble une chaîne alkylène en C₂-C₇ éventuellement substituée une à quatre fois par des groupements alkyle en C₁-C₄ et éventuellement interrompue par des atomes d'oxygène, d'azote ou de soufre,
à partir de diacétylène et d'amines secondaires, caractérisé en ce que
a) l'on sépare par absorption, du gaz de craquage provenant de la production d'acétylène, un courant partiel qui contient du diacétylène de formule II
H-C≡C-C≡C-H (II)
b) on met en réaction ce courant partiel avec des amines secondaires de formule générale III 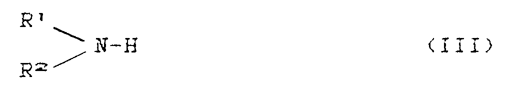 dans laquelle R¹ et R² ont les significations données ci-dessus, et avec de l'eau, eventuellement dans un diluant, à des températures de 0 à 150°C et sous des pressions de 0,01 à 5 bar.

2. Procédé de préparation de 1-aminovinylméthylcétones I selon la revendication 1, caractérisé en ce que l'on effectue l'absorption de manière réversible dans un liquide polaire et on dilue éventuellement avec un gaz inerte.

3. Procédé de préparation de 1-aminovinylméthylcétones selon la revendication 1, caractérise en ce que la part de diacétylène dans le courant partiel dilué, lors de la réaction avec les amines secondaires III, s'élève à 2-30% en volume.
